# EUROPEAN PATENT APPLICATION

(11) **EP 4 752 151 A1**
(43) Date of publication of application: **03.06.2026**
(21) Application number: 24844873.0
(22) Date of filing: 26.07.2024
(51) Int. Cl.: C07G 1/00, C08G 59/06, C08H 7/00, C08J 11/10

(54) **METHOD FOR CATALYTIC DEPOLYMERIZATION OF LIGNIN DERIVATIVE AND LIGNIN DEPOLYMERIZATION OIL**

(30) Priority: 27.07.2023 CN 202310931641
(71) Applicant: Dalian Institute Of Chemical Physics, Chinese Academy Of Sciences, Liaoning 116023 (CN)
(72) Inventor: WANG, Feng, Dalian, Liaoning 116023 (CN); LI, Ning, Dalian, Liaoning 116023 (CN); LIU, Huifang, Dalian, Liaoning 116023 (CN); YAN, Kexin, Dalian, Liaoning 116023 (CN)
(74) Representative: ZHAOffice SPRL
(86) International application number: PCT/CN2024/107769
(87) International publication number: WO 2025/021187

(57) **Abstract**

Disclosed in the present invention are a method for catalytic depolymerization of a lignin derivative, and a lignin depolymerization oil. The method relates to the preparation of a lignin depolymerization oil rich in bisphenols with a 1,2-diphenylethane structure and lignin oligomers by performing synergistic catalysis of a transition metal catalyst, a hydrogen donor and an acidic catalyst on a lignin derivative containing a 1,1-diphenylmethane structure in a liquid medium. The present invention achieves directional depolymerization of lignin by catalyzing an aryl migration reaction of the 1,1-diphenylmethane structure. Different from traditional reactions with lignin monophenols as target products, the present invention can achieve the directional preparation of bisphenol products in one step, and co-production of the lignin oligomer at the same time. The method involved in the present invention expands the product types of lignin-based bisphenols, and facilitates the applications of catalytically depolymerizing lignin to prepare high-value chemicals, fuels and polymer materials.

## Description

### TECHNICAL FIELD

The present invention relates to a method for catalytic depolymerization of a lignin derivative and a lignin depolymerization oil, and particularly relates to the high-valued utilization technology of catalytic depolymerization of lignin and biomass using transition metal catalysts, directed to lignin derivatives having a 1,1-diphenylmethane structure.

### BACKGROUND

Lignin is a natural aryl polymer with great application potential. Due to its advantages such as extensive sources and low costs, lignin is an important potential raw material for the preparation of green aryl chemicals. Lignin is mainly composed of structural units such as p-hydroxyphenyl, guaiacyl, and syringyl. Although the linkage forms between these structural units are diverse, these structural units are mainly connected by C-O bonds, e.g., β-O-4 aryl ether bonds. By catalytically cleaving the C-O bonds in lignin, lignin may be depolymerized into monophenolic compounds such as phenol, guaiacol, propylguaiacol, syringol, propylsyringol, vanillin, syringic aldehyde, and the like. Currently, there are no direct utilization pathways for lignin monophenols. Through subsequent catalytic refining processes, lignin monophenols may be converted into high value-added pharmaceutical intermediates, lignin-based fuels, and the like.

Bisphenol A (BPA) and other bisphenol polymer monomers are the main utilization modes of petrochemical-based phenol, which are mainly prepared by the condensation reaction of two molecules of phenol and one molecule of acetone. At present, lignin monophenols may also be crosslinked with formaldehyde, or condensed between monophenols, to prepare lignin-based bisphenols (US11414519B2; Green Chem., 2018, 20, 1050-1058; Green Chem., 2019, 21, 6622-6633). Such lignin-based bisphenols may meet the synthesis requirements of thermoplastic and thermosetting polymers. Compared with petrochemical-based BPA, their endocrine-disrupting toxicity is significantly reduced. Therefore, lignin-based bisphenols are expected to become important structural monomers for the synthesis of green and renewable polymer materials.

Currently, it is difficult to directly prepare lignin-based bisphenols by catalytic depolymerization of lignin. Taking natural lignin as an example, the content of β-O-4 aryl ether bonds between its structural units is higher than 50%, while the relative content of C-C bonds such as β-β, β-5, and β-5 between structural units is lower, and their reactivity is poor *(*Nat. Chem., 2021, 13, 1118-1125). Therefore, after catalytic depolymerization of natural lignin, the yield of bisphenols is low (less than 5%), and the product structure is complex, thereby making it difficult to generate practical utilization value. Taking industrial lignin as an example, during chemical processing, random condensation and crosslinking reactions readily occur between and within lignin molecular chains, to form a large number of low-activity C-C bond linkages. Therefore, after catalytic depolymerization of industrial lignin, the C-C bonds generated by self-condensation of lignin are difficult to break, resulting in oligomers as the main products. Hence, it is difficult to generate monophenol or bisphenol products (CN202111477348.6).

### SUMMARY

The objective of the present invention is to provide a technical route for preparing a lignin-based bisphenol by catalytic depolymerization of a lignin derivative having a 1,1-diphenylmethane structure, so as to break through the limitation that the major products of lignin depolymerization are monophenolic compounds.

The specific technical solution of the present invention is as follows: a lignin derivative having a 1,1-diphenylmethane structure, a transition metal catalyst, a liquid solvent, an acidic catalyst, and a hydrogen donor are added to a high-temperature and high-pressure reaction kettle in a certain proportion. After sealing the reaction kettle, the mixture is stirred at a constant speed of 600 rpm, heated up to the reaction temperature, maintained at the reaction temperature for a certain period, and then the reaction kettle is cooled. The reaction mixture is subjected to suction filtration, distilled and concentrated under reduced pressure, and extracted to obtain the lignin depolymerization oil rich in lignin-based bisphenols and oligomers.

In the present invention, the lignin derivative having a 1,1-diphenylmethane structure is prepared by directionally substituting benzylic hydroxyl in a β-O-4 aryl ether bond structure of a raw material lignin with phenol and its derivatives. The aromatic ring of the arylation reagent, in addition to containing a hydroxyl group, further contains 0, 1, or 2 substituents. The substituents are any one or a mixture of two or more of methyl, ethyl, propyl, isopropyl, methoxy, phenoxy, or phenolic hydroxyl, and preferably, the arylation reagent is any one or a mixture of two or more of phenol, p-cresol, guaiacol, or syringol.

In the present invention, the lignin derivative having a 1,1-diphenylmethane structure is prepared by the following process:
40 g of lignocellulose, 16 g to 32 g of the arylation reagent, and 400 mL of 88 wt% aqueous organic acid solution are weighed and added to a reaction kettle. The reaction kettle is sealed, and heated up to 100°C under stirring for reacting for 120 min. After cooling, a reaction mixture is filtered and a filtrate is collected for concentration. A concentrated solution is mixed with an antisolvent (water or aqueous ethanol, and a volume fraction of ethanol in the antisolvent being 0%-20%) to precipitate arylated lignin via the antisolvent. The arylated lignin is filtered and dried to obtain arylated lignin powder. The lignocellulose is any one or a mixture of two or more of poplar, birch, pine, bamboo, crop straw, and reed. The arylation reagent is any one or a mixture of two or more of phenol, p-cresol, guaiacol, and syringol. The organic acid is any one or a mixture of two or more of formic acid, acetic acid, maleic acid, oxalic acid, and p-toluenesulfonic acid.

In the present invention, the catalytic depolymerization solvent system for the lignin derivative is any one or a mixture of two or more of methanol, ethanol, propanol, butanol, isopropanol, dioxane, tetrahydrofuran, and water. The preferred lignin solvent is a methanol/water mixture having a volume ratio of 4:1 to 1:4, more preferably 3:1 to 1:1. The mass of the liquid medium is 5-500 times the mass of the lignin derivative, preferably 10-100 times, and more preferably 20-50 times the mass of the lignin derivative.

In the present invention, the acidic catalyst in the catalytic depolymerization system for the lignin derivative is any one or a mixture of two or more of hydrochloric acid, sulfuric acid, phosphoric acid, nitric acid, p-hydroxybenzenesulfonic acid, p-toluenesulfonic acid, methanesulfonic acid, trifluoromethanesulfonic acid, trifluoroacetic acid, phosphotungstic acid, phosphomolybdic acid, zinc chloride, ferric chloride, cupric chloride, aluminum chloride, and lithium chloride. Preferably, the acidic catalyst is any one or a mixture of two or more of sulfuric acid, p-toluenesulfonic acid, and methanesulfonic acid. Preferably, the concentration of the acidic catalyst is 0.5-20 g/L, preferably 1-10 g/L, and more preferably 2-5 g/L.

In the present invention, the transition metal catalyst is selected from any one or a mixture of two or more of palladium, platinum, ruthenium, nickel, rhodium, and iridium, and preferably palladium and platinum. The carrier selected for the transition metal catalyst is any one or a mixture of two or more of activated carbon, alumina, silica, titania, zirconia, ceria, molybdenum oxide, molybdenum sulfide, tungsten oxide, hydrotalcite, hydroxyapatite, and a molecular sieve (H-ZSM-5), and preferably, the catalyst carrier is any one or a mixture of two or more of activated carbon, titania, molybdenum oxide, and molybdenum sulfide. The loading amount of the transition metal on the carrier is 0.1-15 wt%, preferably 0.5-10 wt%, and more preferably 2-5 wt%. The amount of the transition metal catalyst added is 2-200 wt%, preferably 10-80 wt%, and more preferably 20-50 wt% of the mass of the lignin derivative.

In the present invention, the hydrogen donor for the catalytic reduction depolymerization is any one or a mixture of two or more of formic acid, methanol, ethanol, isopropanol, glucose, xylose, ethylene glycol, glycerol, and hydrogen, and preferably formic acid, isopropanol, and hydrogen. The preferred amount of the formic acid added (based on the mass of lignin) is 50-400 wt%, the preferred amount of the isopropanol added (based on the mass of lignin) is 100-500 wt%, and the preferred hydrogen pressure is 0.2-5 MPa. More preferably, the amount of the formic acid added (based on the mass of lignin) is 100-300 wt%.

In the present invention, the catalytic depolymerization reaction of the lignin derivative is performed at a reaction temperature of 120-300°C for a reaction time of 0.2-48 h, preferably at a reaction temperature of 160-260°C for a reaction time of 0.5-24 h, and more preferably at a reaction temperature of 180-240°C for a reaction time of 1-12 h.

After catalytic depolymerization, the lignin depolymerization oil is prepared by performing filtration, distillation under reduced pressure, and liquid-liquid extraction separation on the reaction mixture after reaction.

In the present invention, after the catalytic depolymerization is completed and the reaction kettle is cooled to room temperature, the reaction mixture is subjected to suction filtration through filter paper to remove the transition metal catalyst. The volatile solvent in the filtrate is removed by rotary evaporation under reduced pressure. After being concentrated, the filtrate is subjected to liquid-liquid extraction (dichloromethane: water, 1:1, v/v) three times. Residual water in the dichloromethane phase is removed by anhydrous sodium sulfate, and the organic solvent is removed by rotary evaporation under reduced pressure, to prepare lignin depolymerization oil rich in bisphenols.

The present method involves the selective preparation of a lignin-based bisphenol compound having a 1,2-diphenylethane structure by performing synergistic catalysis of a transition metal catalyst and an acidic catalyst on the lignin derivative having a 1,1-diphenylmethane structure in a liquid medium. The present invention achieves directional depolymerization of lignin by catalyzing an aryl migration reaction of the 1,1-diphenylmethane structure. Different from traditional reactions with lignin monophenols are target products, the present invention can achieve the directional preparation of lignin-based bisphenol product in one step, and co-production of the lignin oligomer at the same time. Hence, the present invention thereby effectively promotes the high-value utilization of lignin.

The method involved in the present invention expands the product types of lignin-based bisphenols, and facilitates the uses of catalytically depolymerizing lignin to prepare high-value chemicals, fuels and polymer materials.

Compared with natural lignin (lignin containing a large amount of benzylic hydroxyl), the lignin derivatives involved in the present invention (the benzylic hydroxyl of lignin is substituted by arylation with phenol or phenol derivatives to generate a 1,1-diphenylmethane structure) exhibit significantly reduced activity in the catalytic cleavage of β-O-4 aryl ether bonds. Therefore, lignin having a 1,1-diphenylmethane structure (such as industrial lignin with a self-condensation structure) is generally difficult to achieve high-valued catalytic conversion. The present invention utilizes the synergistic effect of acid catalysis and hydrogenation catalyst to achieve directional catalytic conversion of the 1,1-diphenylmethane structure in lignin. Lignin-based bisphenol products are prepared highly selectively, and lignin oligomers are co-produced. In this process, the lignin derivative is first catalyzed by acidic additives for 1,2-aryl migration, and the β-O-4 aryl ether bond connected to the 1,1-diphenylmethane structure is cleaved to generate a stilbene-type structural intermediate. The stilbene-type structural intermediate is unstable under high-temperature acidic conditions and is prone to double bond coupling reactions. Hence, a hydrogenation catalyst is utilized in the present invention to reduce the stilbene-type structure to a stable 1,2-diphenylethane bisphenol, thereby improving the selectivity and yield of bisphenol products. In the lignin structural units involved in the present invention, the benzylic carbon has undergone arylation, resulting in a significant reduction in benzylic hydroxyl content. Therefore, the yield of monophenols in the catalytic depolymerization process is significantly lower than that in the traditional catalytic reduction depolymerization of natural lignin. The arylated lignin involved in the invention contains a small amount of C-C bonds such as β-β and β-5 between structural units, which are relatively stable and free of cleavage under the catalytic depolymerization conditions of the present invention, so lignin oligomers rich in C-C bonds may be co-produced. The present invention involves the synergistic depolymerization of arylated lignin by acidic additives and transition metal catalysts, which achieves the selective preparation of lignin-based bisphenol products and co-production of lignin oligomers.

Compared with the prior art, the present invention has the following inventive and beneficial effects:
1. Based on the structural features of 1,1-diphenylmethane in arylated lignin, the present invention designs a directional depolymerization pathway involving acidic catalysis and reductive catalysis, and develops a one-pot tandem reaction containing aryl migration and stilbene-type reduction steps. Hence, bisphenol products with a 1,2-diphenylethane structure are prepared. This catalytic conversion route and the 1,2-diphenylethane bisphenol product are original.
2. The present invention achieves the synergistic catalytic depolymerization of arylated lignin by acidic additives and transition metal catalysts, and the yield of lignin-based bisphenol products is significantly higher than that reported in the prior art.

### BRIEF DESCRIPTION OF THE DRAWINGS

FIG. 1 shows ¹H-¹³C HSQC NMR spectra of arylated lignin derived from phenol, guaiacol, syringol, and p-cresol;
FIG. 2 shows gas chromatograms of monophenols and bisphenols generated by catalytic depolymerization of syringol-arylated lignin;
FIG. 3 shows ¹H-¹³C HSQC NMR spectra for four major bisphenols generated by catalytic depolymerization of syringol-arylated lignin;
FIG. 4 shows ¹H-¹³C HSQC NMR spectrum of oligomeric components in lignin depolymerization oil;
FIG. 5 shows gas chromatogram of monophenols and bisphenols generated by catalytic depolymerization of phenol-arylated lignin; and
FIG. 6 shows ¹H-¹³C HSQC NMR spectra for two major bisphenols generated by catalytic depolymerization of phenol-arylated lignin.

### DETAILED DESCRIPTION OF THE EMBODIMENTS

To further elaborate and illustrate the present invention specifically, the following specific embodiments are set forth; however, the present invention is not limited to the embodiments described below.

In the following examples, Pd/C (5% and 10% metal loading), Pt/C catalyst (5% and 10% metal loading), and Ru/C (5% metal loading) were purchased from Aladdin Scientific.

Pd/ZrO₂ (1% metal loading), Pd/WO₃ (1% metal loading), Pd/MoO₃ (1% metal loading), Pd/TiO₂ (2% metal loading), Pd/HZSM-5 (2% metal loading), and Pt/TiO₂ (2% metal loading) were prepared by the impregnation method.

The impregnation method is exemplified by Pd/TiO₂ (2% metal loading): 1 g of TiO₂ (P25) was taken and dispersed into 20 mL of aqueous hydrochloric acid solution dissolved with PdCl₂ (palladium chloride) (pH=1, PdCl₂ concentration: 9.6 mmol/L, calculated as 2% Pd mass loading in the catalyst), stirred at room temperature for 12 h, then heated and evaporated to dryness at 110°C. The resulting solid was reduced in hydrogen at 300°C for 4 h to obtain the catalyst Pd/TiO₂ (2% metal loading). The preparation process for the remaining catalysts is the same as above, except different concentrations of PdCl₂ (e.g., 4.8 mmol/L) and/or different carriers are used, or H₂PtCl₆ is used to replace PdCl₂.

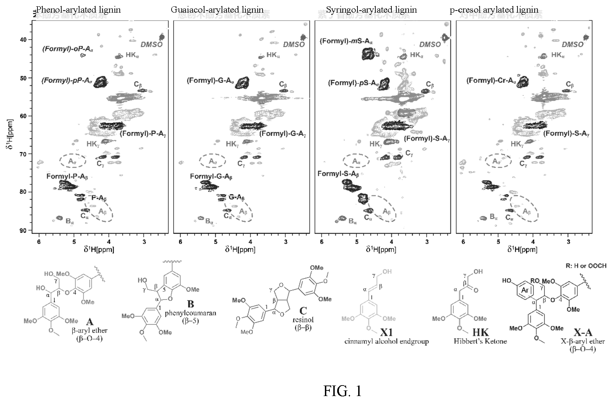

In the following examples of the lignin derivative (rich in a 1,1-diphenylmethane structure), the corresponding arylated lignin is prepared by directionally substituting benzylic hydroxyl in the β-O-4 aryl ether bond structure of lignin raw material with phenol and derivatives thereof. As demonstrated in FIG. 1, in the chemical structure of arylated lignin prepared by the following method, the signal of benzylic hydroxyl groups within the β-O-4 aryl ether bond linkage originally present in natural lignin essentially disappears, replaced by the aryl ether bond signal having a 1,1-diphenylmethane structure generated by the substitution of benzylic hydroxyl with aryl group.

The extraction conditions for the phenol-arylated lignin are as follows: 40 g of birch powder (20-80 meshes), 32 g of phenol, and 400 mL of 88 wt% formic acid solution were weighed and added to a high-temperature reaction kettle. After sealing the reaction kettle, stirring was started at a constant speed of 500 rpm. The reaction kettle was heated up to 100°C for reaction 120 min. After the reaction kettle was cooled to room temperature, the reaction mixture was subjected to suction filtration with filter paper, filtrate was collected and concentrated by rotary evaporation to 150 mL. The concentrated solution was dropped into 1000 mL of water, and arylated lignin dissolved out in a precipitation form. The arylated lignin was collected by filter paper filtration, then dried in an oven at 60°C, and the phenol-arylated lignin powder was collected.

The extraction conditions for the p-cresol arylated lignin are as follows: 40 g of bamboo powder (4-40 meshes), 32 g of p-cresol, and 400 mL of 88 wt% formic acid solution were weighed and added to a high-temperature reaction kettle. After sealing the reaction kettle, stirring was started at a constant speed of 500 rpm. The reaction kettle was heated up to 120°C for reaction 40 min. After the reaction kettle was cooled to room temperature, the reaction mixture was subjected to suction filtration with filter paper, filtrate was collected and concentrated by rotary evaporation to 150 mL. The concentrated solution was dropped into 1000 mL of an antisolvent (water/ethanol, 9:1, v/v), and arylated lignin dissolved out in a precipitation form. The arylated lignin was collected by filter paper, then dried in an oven at 60°C, and the p-cresol arylated lignin powder was collected.

The extraction conditions for the guaiacol-arylated lignin are as follows: 40 g of poplar (4-40 meshes), 32 g of guaiacol, and 400 mL of 88 wt% formic acid solution were weighed and added to a high-temperature reaction kettle. After sealing the reaction kettle, stirring was started at a constant speed of 500 rpm. The reaction kettle was heated up to 120°C for reaction 40 min. After the reaction kettle was cooled to room temperature, the reaction mixture was subjected to suction filtration with filter paper, filtrate was collected and concentrated by rotary evaporation to about 150mL. The concentrated solution was dropped into 1000 mL of an antisolvent (water/ethanol, 9:1, v/v), and arylated lignin dissolved out in a precipitation form. The arylated lignin was collected by filter paper, then dried in an oven at 60°C, and the guaiacol-arylated lignin powder was collected. The preparation conditions for syringol-arylated lignin are as follows: 40 g of poplar (20-80 meshes), 16 g of syringol, and 400 mL of 88 wt% formic acid solution were weighed and added to a high-temperature reaction kettle. After sealing the reaction kettle, stirring was started at a constant speed of 500 rpm. The reaction kettle was heated up to 120°C for reaction 30 min. After the reaction kettle was cooled to room temperature, the reaction mixture was subjected to suction filtration with filter paper, filtrate was collected and concentrated by rotary evaporation to about 150 mL. The concentrated solution was dropped into 1000 mL of water, and arylated lignin dissolved out in a precipitation form. The arylated lignin was subjected to suction filtration with filter paper, then dried in an oven at 60°C, and the syringol-arylated lignin powder was collected.

The extraction conditions for kraft lignin are as follows: the raw material was poplar powder (4-40 meshes); the solution was an aqueous solution containing a final concentration of 10 wt% NaOH and 3 wt% Na₂S, with a liquid to solid mass ratio of 4:1; the cooking temperature was 160°C, and the cooking time was 3 h.

The extraction conditions for enzymatic hydrolysis lignin are as follows: the poplar powder (4-40 meshes) was processed by a planetary ball mill (a ball-to-material ratio of 50:1, a revolving speed of 460 rpm) for 24 h, then treated with commercial cellulase (CTec3, 50 FPU/g wood powder) in an acetic acid/sodium acetate buffer solution (pH=4.8, 20 mL/g wood powder) for 48 h. The enzymatic hydrolysis temperature was 50°C and the oscillation rate was 100 rpm. The undissolved residue was filtered and collected, purified by 90 wt% acetic acid solution (20 mL/g residue) at room temperature, then lignin was precipitated in water (the volume was 10 times that of the purified acetic acid solution).

**Example 1.** 1 g of syringol-arylated lignin, 0.5 g of Pd/C (Pd content in Pd/C was 5 wt% loading, same below), 50 g of mixed solution of methanol and water (7:3, v/v) (containing 1 g/L of p-toluenesulfonic acid), and 200 wt% (the amount added was based on the mass of lignin) of formic acid were weighed and added to a high-temperature and high-pressure reaction kettle. After sealing the reaction kettle, argon was introduced to purge the reaction kettle headspace, stirring was started at a constant speed (600 rpm). The reaction kettle was heated up to 200°C for reaction for 24 h. After the reaction kettle was cooled (to room temperature, same below), the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols.

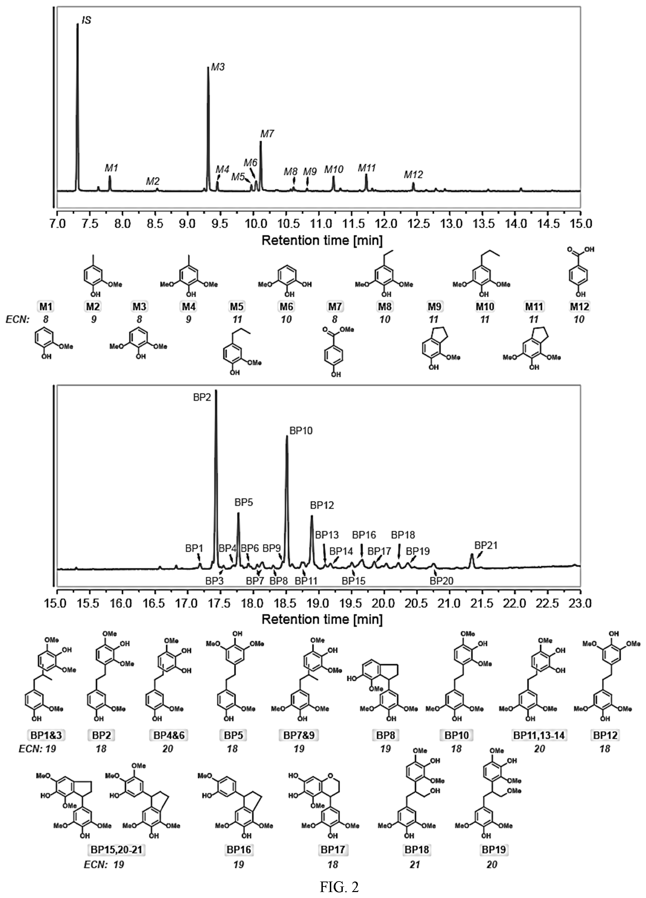

The yields of monophenols (mainly including phenol, guaiacol, 4-alkylguaiacol, syringol, 4-alkylsyringol, etc., where the alkyl carbon number does not exceed 3) and bisphenols (mainly including 3-(4-hydroxy-3-methoxyphenethyl)-2,6-dimethoxyphenol, 4-(4-hydroxy-3-methoxyphenethyl)-2,6-dimethoxyphenol, 3-(4-hydroxy-3,5-dimethoxyphenethyl)-2,6-dimethoxyphenol, 4-(4-hydroxy-3,5-dimethoxyphenethyl)-2,6-dimethoxyphenol, etc.) in the lignin depolymerization oil were quantitatively analyzed by Agilent 7890A gas chromatography. The gas chromatogram is shown in FIG. 2. The yield of the lignin depolymerization oil is the percentage of the mass of the lignin depolymerization oil to the mass of the arylated lignin (raw material). The yield of lignin oligomers (mainly oligomeric lignin products with molecular weights of about 400-900 g/mol, where the aryl units of the lignin oligomers are primarily connected by C-C bonds) was calculated by subtracting the total yields of monophenols and bisphenols from the yield of the lignin depolymerization oil.

**Example 2.** 1 g of syringol-arylated lignin, 0.5 g of Pd/C (5 wt% metal loading), 50 g of mixed solution of methanol and water (7:3, v/v) (containing 2 g/L of p-toluenesulfonic acid), and 200 wt% of (the amount added was based on the mass of lignin) formic acid were weighed and added to a high-temperature and high-pressure reaction kettle. After sealing the reaction kettle, argon was introduced to purge the reaction kettle headspace, stirring was started at a constant speed (600 rpm). The reaction kettle was heated up to 180°C for reaction for 12 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

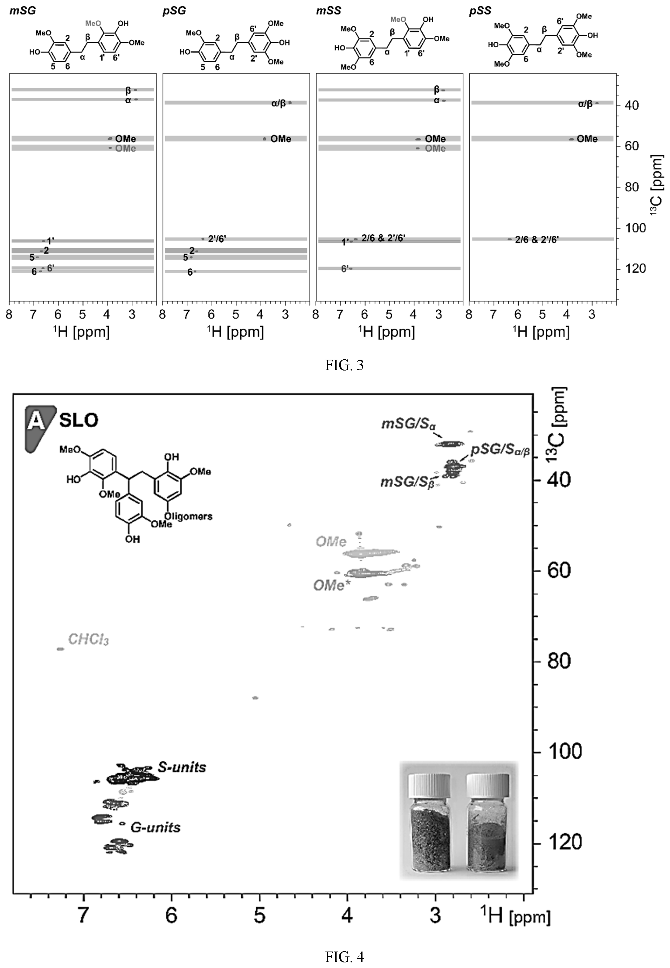

The sample of lignin depolymerization oil was separated by a preparative chromatography column (stationary phase: 200-300 meshes of silica gel; eluent: a mixed solution of ethyl acetate and petroleum ether in a volume ratio of 1:4). The 2D NMR spectra of the four major bisphenols are shown in FIG. 3. The results indicate that the lignin-based bisphenol has an ethylene-bridged structure. The BP2 bisphenol crystal and terephthaloyl chloride may be synthesized into polyarylate plastics according to the method described in the literature (GreenChem., 2018, 20, 1050-1058). The polymer has a weight-average molecular weight of approximately 30 kg/mol, and exhibits good thermal stability (T_{d5%} = 390°C, T_{dmax} = 425°C).

The sample of lignin depolymerization oil was separated into monophenol and bisphenol components and lignin oligomer components by solvent reflux extraction. Solvent reflux extraction was performed using a Soxhlet extractor, with n-hexane as the solvent and a reflux temperature of 100°C. The 2D NMR spectra of the lignin oligomer components are shown in FIG. 4. The results indicate that the aryl structural units within the lignin oligomer components are mainly connected by C-C bonds, and the chemical structure exhibits good rigidity; an epoxy resin with a rigid backbone structure may be prepared via epoxidation reaction.

**Example 3.** 1 g of syringol-arylated lignin, 0.5 g of Pd/C (5 wt% metal loading), 50 g of mixed solution of methanol and water (7:3, v/v) (containing 5g/L of p-toluenesulfonic acid), and 200 wt% of (the amount added was based on the mass of lignin) formic acid were weighed and added to a high-temperature and high-pressure reaction kettle. After sealing the reaction kettle, argon was introduced to purge the reaction kettle headspace, stirring was started at a constant speed (600 rpm). The reaction kettle was heated up to 200°C for reaction for 6 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Example 4.** 1 g of syringol-arylated lignin, 0.5 g of Pd/C (5 wt% metal loading), 50 g of mixed solution of methanol and water (7:3, v/v) (containing 8g/L of p-toluenesulfonic acid), and 200 wt% of (the amount added was based on the mass of lignin) formic acid were weighed and added to a high-temperature and high-pressure reaction kettle. After sealing the reaction kettle, argon was introduced to purge the reaction kettle headspace, stirring was started at a constant speed (600 rpm). The reaction kettle was heated up to 200°C for reaction for 4 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Example 5.** 1 g of syringol-arylated lignin, 0.5 g of Pd/C (5 wt% metal loading), 50 g of mixed solution of methanol and water (7:3, v/v) (containing 10g/L of p-toluenesulfonic acid), and 200 wt% of (the amount added was based on the mass of lignin) formic acid were weighed and added to a high-temperature and high-pressure reaction kettle. After sealing the reaction kettle, argon was introduced to purge the reaction kettle headspace, stirring was started at a constant speed (600 rpm). The reaction kettle was heated up to 200°C for reaction for 4 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Example 6.** 1 g of syringol-arylated lignin, 0.5 g of Pd/C (5 wt% metal loading), 50 g of mixed solution of methanol and water (7:3, v/v) (containing 10g/L of p-toluenesulfonic acid), and 200 wt% of (the amount added was based on the mass of lignin) formic acid were weighed and added to a high-temperature and high-pressure reaction kettle. After sealing the reaction kettle, argon was introduced to purge the reaction kettle headspace, stirring was started at a constant speed (600 rpm). The reaction kettle was heated up to 160°C for reaction for 24 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Example 7.** 2 g of p-cresol arylated lignin, 1 g of Pd/ZrO₂ (Pd content in Pd/ZrO₂ was 1 wt% loading amount), 50 g of mixed solution of methanol and water (2:1, v/v) (containing 5 g/L of p-toluenesulfonic acid), and 200 wt% of formic acid (the amount added was based on the mass of lignin) were weighed and added to a high-temperature and high-pressure reaction kettle. After sealing the reaction kettle, argon was introduced to purge the reaction kettle headspace, stirring was started at a constant speed (600 rpm). The reaction kettle was heated up to 200°C for reaction for 4 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Example 8.** 2 g of p-cresol arylated lignin, 1 g of Pd/WO₃ (1 wt% metal loading, 50 g of mixed solution of methanol and water (2:1, v/v) (containing 10 g/L of hydrochloric acid), and 200 wt% of formic acid (the amount added was based on the mass of lignin) were weighed and added to a high-temperature and high-pressure reaction kettle. After sealing the reaction kettle, argon was introduced to purge the reaction kettle headspace, stirring was started at a constant speed (600 rpm). The reaction kettle was heated up to 220°C for reaction for 8 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Example 9.** 1 g of phenol-arylated lignin, 0.5 g of Pd/MoO₃ (Pd content in Pd/MoO₃ was 1 wt% loading amount), 50 g of mixed solution of methanol and water (2:1, v/v) (containing 10 g/L of phosphoric acid), and 200 wt% of formic acid (the amount added was based on the mass of lignin) were weighed and added to a high-temperature and high-pressure reaction kettle. After sealing the reaction kettle, argon was introduced to purge the reaction kettle headspace, stirring was started at a constant speed (600 rpm). The reaction kettle was heated up to 240°C for reaction for 2 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Example 10.** 1 g of phenol-arylated lignin, 0.5 g of Pd/TiO₂ (2 wt% metal loading), 50 g of mixed solution of methanol and water (2:1, v/v) (containing 5 g/L of sulfuric acid), and 200 wt% of formic acid (the amount added was based on the mass of lignin) were weighed and added to a high-temperature and high-pressure reaction kettle. After sealing the reaction kettle, argon was introduced to purge the reaction kettle headspace, stirring was started at a constant speed (600 rpm). The reaction kettle was heated up to 200°C for reaction for 4 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Example 11.** 1 g of phenol-arylated lignin, 0.5 g of Pd/H-ZSM-5 (Pd content in Pd/H-ZSM-5 was 2 wt% loading amount), 50 g of mixed solution of methanol and water (2:1, v/v) (containing 1 g/L of hydrochloric acid), and 200 wt% of formic acid (the amount added was based on the mass of lignin) were added to a high-temperature and high-pressure reaction kettle. After sealing the reaction kettle, argon was introduced to purge the reaction kettle headspace, stirring was started at a constant speed (600 rpm). The reaction kettle was heated up to 240°C for reaction for 4 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Example 12.** 1 g of phenol-arylated lignin, 0.5 g of Ru/C (5 wt% metal loading), 50 g of mixed solution of methanol and water (2:1, v/v) (containing 10 g/L of hydrochloric acid), and 200 wt% of formic acid (the amount added was based on the mass of lignin) were weighed and added to a high-temperature and high-pressure reaction kettle. After sealing the reaction kettle, argon was introduced to purge the reaction kettle headspace, stirring was started at a constant speed (600 rpm). The reaction kettle was heated up to 220°C for reaction for 8 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Example 13.** 2 g of phenol-arylated lignin, 1 g of Pt/C (5 wt% metal loading), 50 g of mixed solution of methanol and water (4:1, v/v) (containing 5g/L of hydrochloric acid), and 200 wt% of formic acid (the amount added was based on the mass of lignin) were weighed and added to a high-temperature and high-pressure reaction kettle. After sealing the reaction kettle, argon was introduced to purge the reaction kettle headspace, stirring was started at a constant speed (600 rpm). The reaction kettle was heated up to 180°C for reaction for 4 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Example 14.** 1 g of phenol-arylated lignin, 0.5 g of Pt/C (5 wt% metal loading), 50 g of mixed solution of methanol and water (3:1, v/v) (containing 5g/L of hydrochloric acid), and 200 wt% of formic acid (the amount added was based on the mass of lignin) were weighed and added to a high-temperature and high-pressure reaction kettle. After sealing the reaction kettle, argon was introduced to purge the reaction kettle headspace, stirring was started at a constant speed (600 rpm). The reaction kettle was heated up to 200°C for reaction for 8 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Example 15.** 1 g of phenol-arylated lignin, 0.5 g of Pt/TiO₂ (Pt content in Pt/TiO₂ was 2 wt% loading amount), 50 g of mixed solution of methanol and water (1:1, v/v) (containing 5 g/L of sulfuric acid), and 200 wt% of formic acid (the amount added was based on the mass of lignin) were weighed and added to a high-temperature and high-pressure reaction kettle. After sealing the reaction kettle, argon was introduced to purge the reaction kettle headspace, stirring was started at a constant speed (600 rpm). The reaction kettle was heated up to 220°C for reaction for 1 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1. The quantitative analysis of the lignin depolymerization oil is the same as in Example 1.

**Example 16.** 1 g of phenol-arylated lignin, 0.5 g of Pd/C (5 wt% metal loading), 50 g of mixed solution of methanol and water (1:3, v/v) (containing 5 g/L of sulfuric acid), and 50 wt% of formic acid (the amount added was based on the mass of lignin) were weighed and added to a high-temperature and high-pressure reaction kettle. After sealing the reaction kettle, argon was introduced to purge the reaction kettle headspace, stirring was started at a constant speed (600 rpm). The reaction kettle was heated up to 200°C for reaction for 4 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Example 17.** 1 g of phenol-arylated lignin, 0.5 g of Pd/C (5 wt% metal loading), 50 g of mixed solution of methanol and water (1:4, v/v) (containing 5 g/L of sulfuric acid), and 100 wt% of formic acid (the amount added was based on the mass of lignin) were weighed and added to a high-temperature and high-pressure reaction kettle. After sealing the reaction kettle, argon was introduced to purge the reaction kettle headspace, stirring was started at a constant speed (600 rpm). The reaction kettle was heated up to 160°C for reaction for 12 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Example 18.** 1 g of phenol-arylated lignin, 0.5 g of Pd/C (Pd content in Pd/C was 10 wt% loading amount), 50 g of mixed solution of methanol and water (7:3, v/v) (containing 5 g/L of methanesulfonic acid), and 200 wt% of formic acid (the amount added was based on the mass of lignin) were weighed and added to a high-temperature and high-pressure reaction kettle. After sealing the reaction kettle, argon was introduced to purge the reaction kettle headspace, stirring was started at a constant speed (600 rpm). The reaction kettle was heated up to 200°C for reaction for 4 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Example 19.** 5 g of phenol-arylated lignin, 2.5 g of Pd/C (5 wt% metal loading), 50 g of mixed solution of methanol and water (7:3, v/v) (containing 2 g/L of p-toluenesulfonic acid), and 400 wt% of formic acid (the amount added was based on the mass of lignin) were weighed and added to a high-temperature and high-pressure reaction kettle. After sealing the reaction kettle, argon was introduced to purge the reaction kettle headspace, stirring was started at a constant speed (600 rpm). The reaction kettle was heated up to 200°C for reaction for 4 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Example 20.** 2 g of phenol-arylated lignin, 1 g of Pd/C (5 wt% metal loading), 50 g of mixed solution of methanol and water (7:3, v/v) (containing 2 g/L of p-toluenesulfonic acid), and 300 wt% of formic acid (the amount added was based on the mass of lignin) were weighed and added to a high-temperature and high-pressure reaction kettle. After sealing the reaction kettle, argon was introduced to purge the reaction kettle headspace, stirring was started at a constant speed (600 rpm). The reaction kettle was heated up to 200°C for reaction for 4 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Example 21.** 1.5 g of phenol-arylated lignin, 0.75 g of Pd/C (5 wt% metal loading), 50 g of mixed solution of methanol and water (7:3, v/v) (containing 2 g/L of p-toluenesulfonic acid), and 200 wt% of formic acid (the amount added was based on the mass of lignin) were weighed and added to a high-temperature and high-pressure reaction kettle. After sealing the reaction kettle, argon was introduced to purge the reaction kettle headspace, stirring was started at a constant speed (600 rpm). The reaction kettle was heated up to 200°C for reaction for 4 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

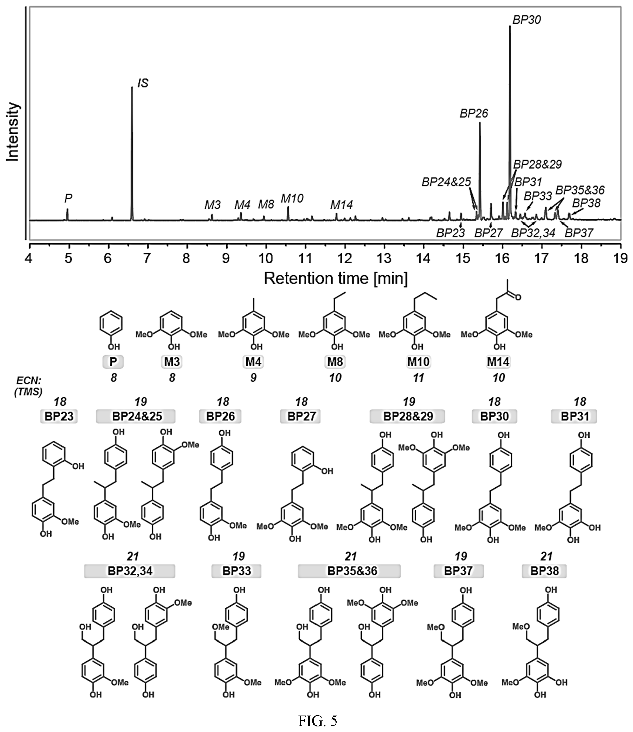

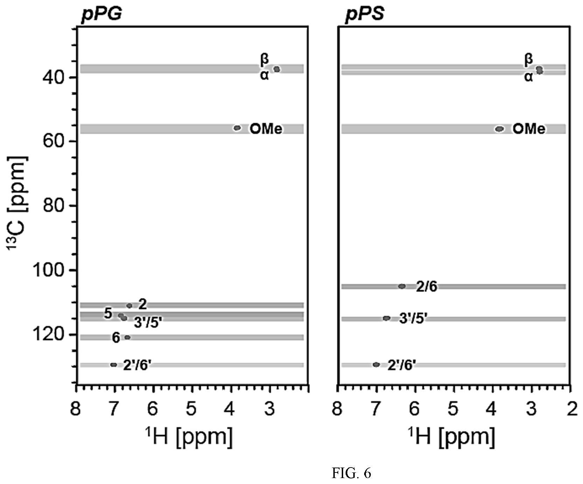

**Example 22.** 1 g of phenol-arylated lignin, 0.5 g of Pd/C (5 wt% metal loading), 50 g of mixed solution of methanol and water (7:3, v/v) (containing 2 g/L of p-toluenesulfonic acid), and 200 wt% of formic acid (the amount added was based on the mass of lignin) were weighed and added to a high-temperature and high-pressure reaction kettle. After sealing the reaction kettle, argon was introduced to purge the reaction kettle headspace, stirring was started at a constant speed (600 rpm). The reaction kettle was heated up to 200°C for reaction for 6 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1. The quantitative analysis of the lignin depolymerization oil is the same as in Example 1. The gas chromatograms of monophenols and bisphenols in the lignin depolymerization oil are shown in FIG. 5. The 2D NMR spectra of the two major bisphenols (4-(4-hydroxyphenethyl)-2-methoxyphenol and 4-(4-hydroxyphenethyl)-2,6-dimethoxyphenol) are shown in FIG. 6. The results indicate that the lignin-based bisphenol has a 1,2-diphenylethane-bridged structure.

**Example 23.** 0.5 g of phenol-arylated lignin, 0.25 g of Pd/C (5 wt% metal loading), 50 g of mixed solution of methanol and water (3:1, v/v) (containing 5 g/L of p-toluenesulfonic acid), and 200 wt% of formic acid (the amount added was based on the mass of lignin) were charged into a high-temperature, high-pressure reaction kettle. After sealing the reaction kettle, argon was introduced to purge the reaction kettle headspace, stirring was started at a constant speed (600 rpm). The reaction kettle was heated up to 200°C for reaction for 4 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Example 24.** 1 g of phenol-arylated lignin, 0.1 g of Pd/C (Pd content in Pd/C was 10 wt% loading amount), 50 g of mixed solution of methanol and water (7:3, v/v) (containing 5 g/L p-toluenesulfonic acid), and 200 wt% of formic acid (the amount added was based on the mass of lignin) were weighed and added to a high-temperature and high-pressure reaction kettle. After sealing the reaction kettle, argon was introduced to purge the reaction kettle headspace, stirring was started at a constant speed (600 rpm). The reaction kettle was heated up to 200°C for reaction for 4 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols.

**Example 25.** 1 g of phenol-arylated lignin, 0.25 g of Pd/C (5 wt% metal loading), 50 g of mixed solution of methanol and water (7:3, v/v) (containing 5 g/L of p-toluenesulfonic acid), and 200 wt% of formic acid (the amount added was based on the mass of lignin) were weighed and added to a high-temperature and high-pressure reaction kettle. After sealing the reaction kettle, argon was introduced to purge the reaction kettle headspace, stirring was started at a constant speed (600 rpm). The reaction kettle was heated up to 200°C for reaction for 4 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Example 26.** 1 g of phenol-arylated lignin, 0.8 g of Pd/C (10 wt% metal loading), 50 g of mixed solution of methanol and water (7:3, v/v) (containing 5 g/L of p-toluenesulfonic acid), and 200 wt% of formic acid (the amount added was based on the mass of lignin) were weighed and added to a high-temperature and high-pressure reaction kettle. After sealing the reaction kettle, argon was introduced to purge the reaction kettle headspace, stirring was started at a constant speed (600 rpm). The reaction kettle was heated up to 200°C for reaction for 4 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Example 27.** 2 g of guaiacol-arylated lignin, 1 g of Pt/C (5 wt% metal loading), 50 g of mixed solution of methanol and water (7:3, v/v) (containing 5 g/L of p-toluenesulfonic acid), and 200 wt% of formic acid (the amount added was based on the mass of lignin) were weighed and added to a high-temperature and high-pressure reaction kettle. After sealing the reaction kettle, argon was introduced to purge the reaction kettle headspace, stirring was started at a constant speed (600 rpm). The reaction kettle was heated up to 200°C for reaction for 4 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Example 28.** 2 g of guaiacol-arylated lignin, 1 g of Pt/C (10 wt% metal loading), 50 g of mixed solution of methanol and water (7:3, v/v) (containing 5 g/L of p-toluenesulfonic acid), and 200 wt% of isopropanol (the amount added was based on the mass of lignin) were weighed and added to a high-temperature and high-pressure reaction kettle. After sealing the reaction kettle, argon was introduced to purge the reaction kettle headspace, stirring was started at a constant speed (600 rpm). The reaction kettle was heated up to 200°C for reaction for 4 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Example 29.** 2 g of guaiacol-arylated lignin, 1 g of Pt/C (10 wt% metal loading), 50 g of mixed solution of methanol and water (7:3, v/v) (containing 5 g/L of p-toluenesulfonic acid), and 400 wt% of isopropanol (the amount added was based on the mass of lignin) were weighed and added to a high-temperature and high-pressure reaction kettle. After sealing the reaction kettle, argon was introduced to purge the reaction kettle headspace, stirring was started at a constant speed (600 rpm). The reaction kettle was heated up to 200°C for reaction for 4 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Example 30.** 1 g of guaiacol-arylated lignin, 0.5 g of Pt/C (5 wt% metal loading), and 50 g of mixed solution of methanol and water (7:3, v/v) (containing 5 g/L of p-toluenesulfonic acid) were weighed and added to a high-temperature and high-pressure reaction kettle. After sealing the reaction kettle, argon was introduced to purge the reaction kettle headspace. Under ambient temperature conditions, 0.5 MPa hydrogen was charged. Stirring was started at a constant speed (600 rpm). The reaction kettle was heated up to 220°C for reaction for 4 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Example 31.** 1 g of guaiacol-arylated lignin, 0.5 g of Pt/C (5 wt% metal loading), and 50 g of mixed solution of methanol and water (7:3, v/v) (containing 5 g/L of p-toluenesulfonic acid) were weighed and added to a high-temperature and high-pressure reaction kettle. After sealing the reaction kettle, argon was introduced to purge the reaction kettle headspace. Under ambient temperature conditions, 2 MPa hydrogen was charged. Stirring was started at a constant speed (600 rpm). The reaction kettle was heated up to 220°C for reaction for 4 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Example 32.** 1 g of syringol-arylated lignin, 0.5 g of Pd/C (5 wt% metal loading), 50 g of mixed solution of methanol and water (7:3, v/v) (containing 5 g/L of tungstic acid), and 200 wt% (the amount added was based on the mass of lignin) of formic acid were weighed and added to a high-temperature and high-pressure reaction kettle. After sealing the reaction kettle, argon was introduced to purge the reaction kettle headspace, stirring was started at a constant speed (600 rpm). The reaction kettle was heated up to 200°C for reaction for 6 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Example 33.** 1 g of syringol-arylated lignin, 0.5 g of Pt/C (5 wt% metal loading), 50 g of mixed solution of methanol and water (7:3, v/v) (containing 5 g/L of tungstic acid), and 200 wt% (the amount added was based on the mass of lignin) of formic acid were weighed and added to a high-temperature and high-pressure reaction kettle. After sealing the reaction kettle, argon was introduced to purge the reaction kettle headspace, stirring was started at a constant speed (600 rpm). The reaction kettle was heated up to 200°C for reaction for 6 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Example 34.** 1 g of phenol-arylated lignin, 0.5 g of Pt/C (5 wt% metal loading), 50 g of a mixed solvent of methanol and water (7:3, v/v), 0.3 g of tungsten trioxide (a solid-acid catalyst), and 200 wt% (the amount added was based on the mass of lignin) of formic acid were weighed and added to a high-temperature and high-pressure reaction kettle. After sealing the reaction kettle, argon was introduced to purge the reaction kettle headspace, stirring was started at a constant speed (600 rpm). The reaction kettle was heated up to 200°C for reaction for 6 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Example 35.** 1 g of phenol-arylated lignin, 0.5 g of Pt/WO₃ (1 wt% metal loading), 50 g of a mixed solvent of methanol and water (7:3, v/v), and 200 wt% of (the amount added was based on the mass of lignin) formic acid were weighed and added to a high-temperature and high-pressure reaction kettle. After sealing the reaction kettle, argon was introduced to purge the reaction kettle headspace, stirring was started at a constant speed (600 rpm). The reaction kettle was heated up to 200°C for reaction for 6 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil rich in bisphenols. The quantitative analysis of the lignin depolymerization oil is the same as in Example 1.

**Comparative Example** 1. 1 g of kraft lignin, 0.5 g of Pd/C (5 wt% metal loading), 50 g of mixed solution of methanol and water (7:3, v/v) (containing 2 g/L of p-toluenesulfonic acid), and 200 wt% of formic acid (the amount added was based on the mass of lignin) were weighed and added to a high-temperature and high-pressure reaction kettle. After sealing the reaction kettle, argon was introduced to purge the reaction kettle headspace, stirring was started at a constant speed (600 rpm). The reaction kettle was heated up to 200°C for reaction for 6 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil. The quantitative analysis of the lignin depolymerization oil is the same as in Example 1.

**Comparative Example 2.** 1 g of enzymatic lignin, 0.5 g of Pd/C (5 wt% metal loading), 50 g of mixed solution of methanol and water (7:3, v/v) (containing 2 g/L of p-toluenesulfonic acid), and 200 wt% (the amount added was based on the mass of lignin) of formic acid were weighed and added to a high-temperature and high-pressure reaction kettle. After sealing the reaction kettle, argon was introduced to purge the reaction kettle headspace, stirring was started at a constant speed (600 rpm). The reaction kettle was heated up to 200°C for reaction for 6 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil. The quantitative analysis of the lignin depolymerization oil is the same as in Example 1.

**Comparative Example 3.** 1 g of phenol-arylated lignin, 50 g of mixed solution of methanol and water (7:3, v/v) (containing 2 g/L p-toluenesulfonic acid), and 200 wt% (the amount added was based on the mass of lignin) of formic acid were weighed and added to a high-temperature and high-pressure reaction kettle. After sealing the reaction kettle, argon was introduced to purge the reaction kettle headspace, stirring was started at a constant speed (600 rpm). The reaction kettle was heated up to 200°C for reaction for 6 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin-rich depolymerization oil. The quantitative analysis of the lignin depolymerization oil is the same as in Example 1.

**Comparative Example 4.** 1 g of phenol-arylated lignin, 0.5 g of Pd/C (5 wt% metal loading), 50 g of mixed solution of methanol and water (7:3, v/v), and 200 wt% (the amount added was based on the mass of lignin) of formic acid were weighed and added to a high-temperature and high-pressure reaction kettle. After sealing the reaction kettle, argon was introduced to purge the reaction kettle headspace, stirring was started at a constant speed (600 rpm). The reaction kettle was heated up to 200°C for reaction for 6 h. After the reaction kettle was cooled, the reaction mixture was subjected to suction filtration with filter paper. The filtrate was concentrated by rotary evaporation under reduced pressure (at about 40°C), then extracted three times with a 250 mL separatory funnel (dichloromethane: water = 1:1, v/v, 100 mL each time). The dichloromethane phase was dried with anhydrous sodium sulfate, and the organic solvent in the dichloromethane phase was removed by rotary evaporation under reduced pressure (at about 40°C) to prepare lignin depolymerization oil. The quantitative analysis of the lignin depolymerization oil is the same as in Example 1.

**Table 1. Preparation of monophenols, bisphenols, and lignin oligomers via the catalytic depolymerization of lignin samples**

| Example No. | Monophenol yield [wt%] | Bisphenol yield [wt%] | Oligomer yield [wt%] |
|---|---|---|---|
| Example 1 | 5.1 | 16.2 | 37.0 |
| Example 2 | 7.7 | 18.6 | 50.5 |
| Example 3 | 9.1 | 19.2 | 49.7 |
| Example 4 | 9.5 | 18.2 | 46.5 |
| Example 5 | 9.8 | 18.1 | 47.2 |
| Example 6 | 8.5 | 17.0 | 36.6 |
| Example 7 | 6.2 | 15.1 | 50.7 |
| Example 8 | 5.9 | 14.2 | 53.9 |
| Example 9 | 1.9 | 17.5 | 45.6 |
| Example 10 | 1.6 | 18.9 | 42.5 |
| Example 11 | 0.9 | 11.3 | 37.8 |
| Example 12 | 0.9 | 12.1 | 44.0 |
| Example 13 | 1.8 | 20.3 | 37.9 |
| Example 14 | 1.9 | 16.4 | 45.7 |
| Example 15 | 1.8 | 21.1 | 44.1 |
| Example 16 | 1.6 | 15.2 | 29.2 |
| Example 17 | 1.4 | 13.5 | 25.1 |
| Example 18 | 1.9 | 24.1 | 46.0 |
| Example 19 | 1.8 | 22.7 | 46.5 |
| Example 20 | 2.0 | 24.3 | 47.9 |
| Example 21 | 2.1 | 26.2 | 46.6 |
| Example 22 | 2.0 | 24.0 | 42.6 |
| Example 23 | 2.2 | 24.8 | 38.0 |
| Example 24 | 2.3 | 23.7 | 43.5 |
| Example 25 | 2.3 | 24.2 | 43.7 |
| Example 26 | 2.4 | 24.6 | 35.0 |
| Example 27 | 4.6 | 18.4 | 44.0 |
| Example 28 | 4.8 | 15.8 | 44.4 |
| Example 29 | 4.7 | 16.8 | 43.5 |
| Example 30 | 4.1 | 13.2 | 33.7 |
| Example 31 | 4.1 | 16.2 | 37.7 |
| Example 32 | 3.2 | 20.2 | 42.7 |
| Example 33 | 2.8 | 18.2 | 47.3 |
| Example 34 | 2.1 | 16.9 | 45.9 |
| Example 35 | 2.5 | 14.5 | 40.8 |
| Comparative Example 1 | 1.3 | 0.3 | 31.0 |
| Comparative Example 2 | 35.9 | 2.1 | 31.5 |
| Comparative Example 3 | 2.1 | 1.5 | 38.4 |
| Comparative Example 4 | 0.2 | 0.1 | 10.0 |

| | | | |
|---|---|---|---|
| Note: all the yields are calculated based on the mass of the arylated lignin samples or extracted lignin samples. | | | |

As shown in Table 1, the method of catalytic depolymerization of lignin derivatives containing 1,1-diphenylmethane structures according to the present invention may effectively reduce the yield of monophenols, selectively produce lignin-based bisphenols, and co-produces lignin oligomers at the same time. Under the catalytic depolymerization conditions described in the present invention, the arylated lignin prepared from syringol, p-cresol, phenol, and guaiacol may undergo depolymerization to efficiently generate bisphenols containing 1,2-diphenylethane structures. Compared with Comparative Example 1 (kraft lignin) and Comparative Example 2 (enzymatic hydrolysis lignin), in Example 2 (syringol-arylated lignin), the lignin-based bisphenol yield increases by 910%-6400% under the same conditions of catalytic depolymerization; in Example 22 (phenol-arylated lignin), the lignin-based bisphenol yield increases by 1100%-8000% under the same conditions of catalytic depolymerization.

In the catalytic depolymerization process described in the present invention, the acidic catalyst and transition metal catalyst are the key factors to affect the formation of bisphenols from lignin derivatives containing 1,1-diphenylmethane structures via catalytic depolymerization. As can be seen from Comparative Example 3, when no transition metal catalyst is present during catalytic depolymerization, the yield of lignin monophenols does not change significantly, but the yield of lignin-based bisphenols drops to 1.5%. The main reason is that the bisphenol products generated by depolymerization under acidic conditions (due to the presence of double bonds) are unstable, and the bisphenol yield reduces significantly due to condensation reaction. As can be seen from Comparative Example 4, when no acidic catalyst is present during catalytic depolymerization, the yields of lignin monophenols, bisphenols, and oligomers all decrease significantly. The main reason is that the transition metal catalyst may not depolymerize the lignin derivative having a 1,1-diphenylmethane structure under neutral conditions. The above results indicate that the method for catalytic depolymerization of lignin derivatives may achieve the directional preparation of bisphenol compounds in one step, and co-production of the lignin oligomer at the same time, thereby promoting the industrial application of lignin catalytic depolymerization for the preparation of high-valued chemicals, fuels, and polymer materials.

The above embodiments are preferred implementations of the present invention, but the embodiments of the present invention are not limited to the above examples. Any other changes, modifications, substitutions, combinations, or simplifications made without departing from the basic chemical reaction principles of the present invention shall be equivalent modes of substitution and are included within the scope of protection of the present invention.

## Claims

1. A method for catalytic depolymerization of a lignin derivative, wherein the lignin derivative having a 1,1-diphenylmethane structure is subjected to synergistic catalysis by a transition metal catalyst, a hydrogen donor, and an acidic catalyst in a liquid medium to prepare a lignin depolymerization oil rich in a bisphenol with a 1,2-diphenylethane structure and a lignin oligomer.

2. The method according to claim 1, wherein the lignin derivative having a 1,1-diphenylmethane structure is a selectively α-arylation modified lignin (as shown in a raw material structural formula on a left side of Formula 1); in a structure of the lignin derivative (as shown in the raw material structural formula on the left side of Formula 1), Ar1 is an aromatic ring originally present in lignin, containing 0, 1, or 2 methoxy groups in an ortho-position of an aromatic ring hydroxyl group on the aromatic ring; Ar2 is an aromatic ring of an arylation reagent, further containing 0, 1, or 2 substituents in addition to a hydroxyl group on the aromatic ring, wherein the substituents are any one or a mixture of two or more of methyl, ethyl, propyl, isopropyl, methoxy, phenoxy, and phenolic hydroxyl, and preferably, the arylation reagent is any one or more of phenol, p-cresol, guaiacol, and syringol; and R₁ is hydroxyl or acyl; 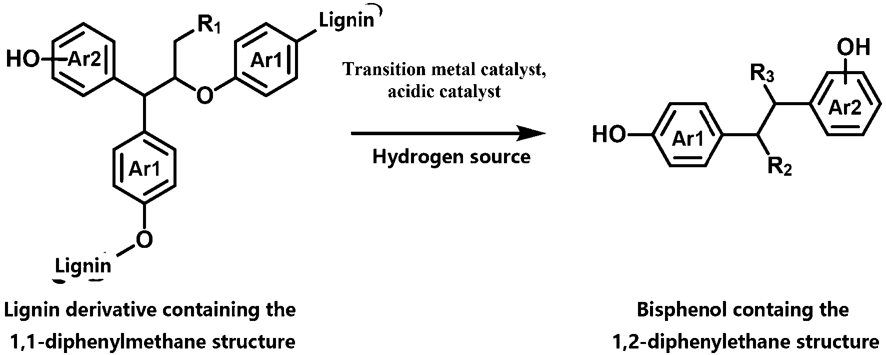
Formula 1. Schematic reaction formula for catalytic depolymerization of the lignin derivative having the 1,1-diphenylmethane structure to prepare a bisphenol product with the 1,2-diphenylethane structure
in the bisphenol product with a 1,2-diphenylethane structure (as shown in a product structural formula on a right side of Formula 1), wherein an ethyl-bridged structure is present between an aromatic ring Ar1 and an aromatic ring Ar2, and substituents R₂ and R₃ denote hydrogen, methyl, or hydroxymethyl; Ar1 is an aromatic ring originally present in lignin, containing 0, 1, or 2 methoxy groups at an ortho-position of an phenolic hydroxyl group on the aromatic ring; Ar2 is an aromatic ring of an arylation reagent, further containing 0, 1, or 2 substituents in addition to a hydroxyl group, wherein the substituents are any one or a mixture of two or more of methyl, ethyl, propyl, isopropyl, methoxy, phenoxy, and phenolic hydroxyl, and preferably, the arylation reagent is any one or more of phenol, p-cresol, guaiacol, and syringol; and R₁ is a hydroxyl or acyl group.

3. The method according to claim 1, wherein
the lignin derivative having a 1,1-diphenylmethane structure is a selectively α-arylation modified lignin (as shown in a raw material structural formula on a left side of Formula 1), and is prepared by directionally substituting benzylic hydroxyl in β-O-4 aryl ether bond structure of a raw material lignin with an arylation reagent, and retaining the β-O-4 aryl ether bond structure of natural lignin (as shown in Formula 2);
in a structure of the lignin derivative (as shown in a raw material structural formula on a left side of Formula 1), Ar1 is an aromatic ring originally present in lignin, containing 0, 1, or 2 methoxy groups in an ortho-position of an aromatic ring hydroxyl group on the aromatic ring; Ar2 is an aromatic ring of an arylation reagent, further containing 0, 1, or 2 substituents in addition to a hydroxyl group on the aromatic ring, wherein the substituents are any one or a mixture of two or more of methyl, ethyl, propyl, isopropyl, methoxy, phenoxy, and phenolic hydroxyl, and preferably, the arylation reagent is any one or more of phenol, p-cresol, guaiacol, and syringol; and R1 is hydroxyl or acyl (the acyl is derived from an organic acid solvent used in a preparation process of the lignin derivative, such as formic acid and acetic acid); 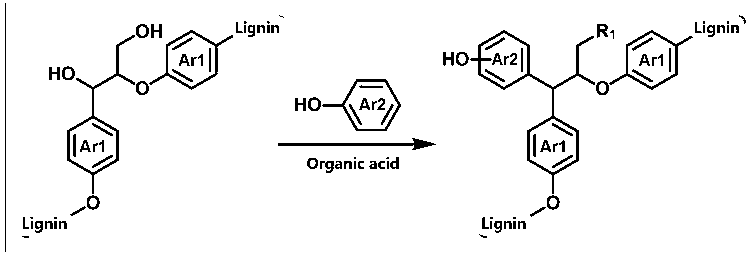 Formula 2. Schematic reaction formula for the preparation of the lignin derivative having the 1,1-diphenylmethane structure by selective α-arylation

4. The method according to claim 1, 2, or 3, wherein
the lignin derivative having a 1,1-diphenylmethane structure is prepared by the following process:
weighing 40-80 g of lignocellulose, 16-40 g of the arylation reagent, and 400 mL of a 60-95 wt% aqueous organic acid solution, and adding to a reaction kettle; after sealing the reaction kettle, heating up to 80-140°C under stirring, and reacting for 30-180 min; after cooling, filtering a reaction mixture and collecting a filtrate for concentration, mixing a concentrated solution with an antisolvent (water or aqueous ethanol, and a volume fraction of ethanol in the antisolvent being 0%-20%) to precipitate arylated lignin via the antisolvent, and filtering and drying the arylated lignin to obtain an arylated lignin powder, wherein
the lignocellulose is any one or a mixture of two or more of poplar, birch, pine, bamboo, crop straw and reed;
the arylation reagent is any one or a mixture of two or more of phenol, cresol, guaiacol, syringol, and catechol; and
the organic acid is any one or a mixture of two or more of formic acid, acetic acid, maleic acid, oxalic acid, and p-toluenesulfonic acid.

5. The method according to claim 1, wherein the liquid medium for a catalytic depolymerization reaction is any one or a mixture of two or more of dioxane, tetrahydrofuran, methanol, ethanol, propanol, butanol, isopropanol, glycerol, toluene, and water; a preferred solvent for the lignin derivative catalytic depolymerization reaction is a methanol/water mixture having a volume ratio of 4:1 to 1:4, and more preferably 3:1 to 1:1; and
the liquid medium has a mass of 5-500 times, preferably 10-100 times, and more preferably 20-50 times a mass of the lignin derivative.

6. The method according to claim 1, wherein the acidic catalyst is any one or a mixture of two or more of hydrochloric acid, sulfuric acid, phosphoric acid, nitric acid, p-hydroxybenzenesulfonic acid, p-toluenesulfonic acid, methanesulfonic acid, trifluoromethanesulfonic acid, trifluoroacetic acid, phosphotungstic acid, phosphomolybdic acid, tungstic acid, tungsten trioxide, zinc chloride, ferric chloride, cupric chloride, aluminum chloride, and lithium chloride, and preferably, the acidic catalyst is any one or a mixture of two or more of sulfuric acid, p-toluenesulfonic acid, and methanesulfonic acid; and the acidic catalyst in the liquid medium has a concentration of 0.5-20 g/L, preferably 1-10 g/L, and more preferably 2-5 g/L.

7. The method according to claim 1, wherein a transition metal selected for the transition metal catalyst in a catalytic depolymerization reaction is any one or a mixture of two or more of palladium, platinum, ruthenium, nickel, rhodium, and iridium; the transition metal catalyst is a supported catalyst, and a carrier selected for the transition metal catalyst is any one or a mixture of two or more of activated carbon, alumina, silica, titania, zirconia, ceria, molybdenum oxide, molybdenum sulfide, tungsten oxide, hydrotalcite, hydroxyapatite, and a molecular sieve (H-ZSM-5); a loading amount of an active component, i.e., the transition metal on the carrier ranges within 0.1-15 wt%, preferably 0.5-10 wt%, and more preferably 2-5 wt%; and
an amount of the transition metal catalyst used ranges within 2-100 wt%, preferably 10-80 wt%, and more preferably 20-50 wt% of the lignin derivative by mass.

8. The method according to claim 1, wherein the hydrogen donor for a catalytic depolymerization reaction is any one or a mixture of two or more of formic acid, methanol, ethanol, isopropanol, glucose, xylose, ethylene glycol, glycerol, and hydrogen; when the hydrogen donor is not hydrogen, an amount of the hydrogen donor used (based on a mass of the lignin derivative) is 50-400 wt%; and when the hydrogen donor is hydrogen, a hydrogen pressure during the reaction is 0.1-8 MPa; and
preferably, the hydrogen donor is any one or more of formic acid, isopropanol, and hydrogen; a preferred amount of the formic acid used (based on a mass of lignin) is 50-400 wt%, a preferred amount of the isopropanol used (based on the mass of the lignin) is 100-500 wt%, and a preferred hydrogen pressure during the reaction is 0.2-5 MPa; and a more preferred amount of the formic acid added (based on the mass of the lignin derivative) is 100-300 wt%.

9. The method according to any one of claims 1-8, wherein the catalytic depolymerization of the lignin derivative is performed at a reaction temperature of 120-300°C for a reaction time of 0.1-48 h, preferably at a reaction temperature of 160-260°C for a reaction time of 0.5-24 h, and more preferably at a reaction temperature of 180-240°C for a reaction time of 1-12 h.

10. A lignin depolymerization oil rich in a bisphenol with a 1,2-diphenylethane structure and lignin oligomers obtained by the method according to any one of claims 1-9.
